# FASCICULE DE BREVET EUROPEEN

(11) **EP 1 680 158 B1**
(45) Date de publication et mention de la délivrance du brevet: **01.07.2015**
(21) Numéro de dépôt: 04787491.2
(22) Date de dépôt: 30.09.2004
(51) Int. Cl.: A61M 5/142, A61M 5/168, A61M 5/172

(54) **DISPOSITIF DE PERFUSION IMPLANTABLE**
IMPLANTIERBARE PERFUSIONSVORRICHTUNG
IMPLANTABLE PERFUSION DEVICE

(30) Priorité: 07.10.2003 FR 0311705
(43) Date de publication de la demande: 19.07.2006
(73) Titulaire: Podvin, Jean-Marie, 59310 Beuvry La Foret (FR)
(72) Inventeur: Podvin, Jean-Marie, 59310 Beuvry la Forêt (FR); Lecoffre, Yves, 38000 Grenoble (FR)
(74) Mandataire: Balesta, Pierre
(86) Numéro de dépôt international: PCT/FR2004/002478
(87) Numéro de publication internationale: WO 2005/035027

(56) Documents cités:
- WO-A2-03/099351
- US-A- 4 077 405
- US-A- 4 714 462
- US-A1- 2002 156 464
- US-B1- 6 264 921

## Description

La présente invention se rapporte aux dispositifs de perfusion de médicaments liquides, implantables, et concerne plus particulièrement, un dispositif implantable pour administration continue de très faibles débits de médicament à un patient.

Un certain nombre de maladies chroniques, telles que le diabète, le cancer, le SIDA, les maladies du sang, exigent que l'on pratique des perfusions de façon continue, dans le but d'administrer au patient une substance médicamenteuse pendant des périodes de temps allant de quelques mois à quelques années.

Plusieurs systèmes de perfusion sont connus actuellement :
- de type fixe, utilisés généralement en milieu hospitalier, où l'écoulement de la substance médicamenteuse se fait par gravitation, par exemple à partir de poches plastiques accrochées au-dessus du lit du patient ; ces systèmes présentent le désavantage majeur d'obliger le patient de rester alité et, par conséquent, limitent fortement leur durée d'utilisation ;
- de type ambulatoire, où l'administration du médicament se fait à l'aide d'une pompe motorisée que le patient porte sur lui, et qui permet l'injection automatique de la substance médicamenteuse dans le corps du patient ; les inconvénients de cette solution résident dans le poids relativement élevé de la pompe, et dans la gêne créée par la tubulure reliant la pompe au site d'injection du médicament, située généralement au niveau de l'abdomen du patient.

De sorte que la solution d'une pompe implantable dans le corps du patient s'impose, grâce aux multiples avantages qu'elle procure :
- niveau élevé de sécurité ;
- plus de confort pour le patient, ce qui lui permet de mener une vie normale ;
- l'assistance médicale dont le patient a besoin est réduite au minimum.

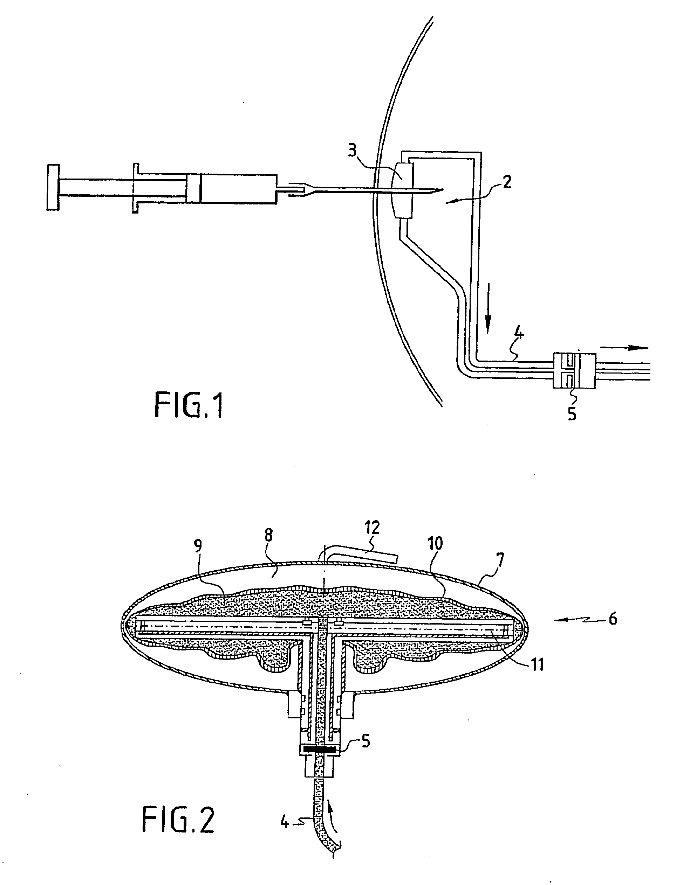

Les dispositifs de perfusion implantables connus dans l'art antérieur comportent une pompe à perfusion comprenant un réservoir à médicament, ladite pompe étant placée sous la peau du patient, dans une poche située généralement dans l'abdomen ; l'orifice de remplissage dudit réservoir est détecté en palpant la peau du patient. Le remplissage dudit réservoir à médicament, sous-pressurisé, se fait périodiquement :
- soit au moyen d'une aiguille reliée à une seringue contenant le médicament liquide ; dans ce cas, l'aiguille traverse la peau du patient, puis l'orifice de remplissage, débouche dans le réservoir et le liquide est délivré par la pression exercée sur la seringue ;
- soit au moyen d'un dispositif de recharge périodique, par exemple une capsule, contenant le liquide à perfuser, qui vient se fixer de manière étanche au niveau de l'orifice de remplissage, au cas où ledit liquide est aspiré dans le réservoir par la dépression existant à l'intérieur de celui-ci. Ce type de dispositif est décrit dans US 2002/0156464 A1 en référence à la figure 1. Ce dispositif implantable comprend un réservoir d'infusion (12, figure 1) destiné à stocker un volume déterminé de liquide et qui peut être rempli via un septum (14, figure 1). Le volume du réservoir est ajusté via des moyens de pression, tel qu'un ressort (16, figure 1). Le réservoir est rempli via une aiguille de remplissage traversant la peau puis le septum. Les documents US 6,264,921 B1, US 4,077,405 B1 et US 4,714,462 B1 ont pour objet des dispositifs implantables configurés comme le dispositif de US 2002/0156464 A1 et dans lesquels le réservoir est directement rempli via une aiguille de remplissage traversant la peau puis le septum (21, figure 1 dans US 6,264,921 B1 ; 16, figure 1 dans US 4,714,462) ou débouchant dans un conduit de remplissage (3, figure 1 dans US 4,077,405 B1).

Ces dispositifs de perfusion implantables connus présentent plusieurs inconvénients :
- l'emplacement sous la peau du patient, dans la région de l'abdomen, peut s'avérer gênant, surtout à long terme ;
- afin de sécuriser au maximum l'opération de remplissage dudit réservoir à médicament, ces dispositifs présentent des structures très complexes, ce qui induit un surcoût de fabrication important ;

- le réservoir doit être sous pressurisé afin d'éviter une fuite du liquide à perfuser dans le corps du patient ;
- le remplissage dudit réservoir à médicament étant une opération très délicate, il nécessite une compétence spéciale de la part du personnel médical chargé de l'effectuer.

Le document WO 03/099351 appartient à l'état de la technique selon l'article 54(3) CBE 1973 pour l'Allemagne, la France, la Grande-bretagne et l'Italie uniquement. Le document WO 03/099351 n'est donc opposable au présent brevet au titre de la nouveauté que pour l'Allemagne, la France, la Grande-bretagne et l'Italie.

Le document WO 03/099351 concerne un dispositif implantable d'administration de médicaments à très faible encombrement qui permet une administration fiable et sûre de doses de médicaments contrôlées sur un site cible. Ce système comprend un réservoir de médicaments à volume variable qui est exposé à une pression ambiante égale à la pression ambiante au niveau d'un orifice d'évacuation du système.

Un sous-ensemble pompe/soupape aspire les médicaments du réservoir et pousse une dose de médicament vers l'orifice d'évacuation le long d'une voie de transfert de fluide. Par ailleurs, la figure 4 représente un dispositif avec un moyen de remplissage 88, apte à être implanté sous la peau et similaire à celui de la figure 1, et un tube de remplissage 90 entre le moyen de remplissage 88 et le réservoir 84.

La présente invention se propose de remédier aux inconvénients précités, en créant un dispositif de perfusion d'un médicament liquide, implantable, comprenant un réservoir de construction simple, qui peut être placé dans une cavité du corps, convenablement choisie pour le confort du patient, la chambre à médicament dudit réservoir étant rechargée périodiquement, de façon simple et sécurisée.

Le dispositif implantable pour administration d'un liquide, notamment d'un médicament, chez l'homme ou l'animal, comprend, selon l'invention :
- un réservoir permettant de délivrer un liquide et destiné à être placé dans une cavité du corps du patient, comprenant :
   - une entrée prévue pour ledit liquide,
   - au moins une sortie pour délivrer ledit liquide au patient ;
- une chambre de transfert permettant l'alimentation d'un réservoir en liquide, apte à être implantée sous la peau, ladite chambre de transfert comprend un orifice de remplissage muni d'un septum, à travers lequel tout moyen approprié peut délivrer ledit liquide ;
- des moyens de transfert dudit liquide entre la chambre de transfert et ledit réservoir.

Par ailleurs, pour l'Allemagne, la France, la Grande-bretagne et l'Italie, le dispositif implantable selon l'invention comprend également un filtre stérile placé en sortie de la chambre de transfert.

Le dispositif de l'invention est destiné à apporter par voie vasculaire centrale une quantité physiologique basale de médicament, notamment d'insuline dans le cas des patients diabétiques insulino-dépendants, de façon continue. Ce dispositif comprend une chambre implantable de dimensions réduites, qui alimente, par un tube sécurisé au moyen d'un clapet anti-retour, un réservoir qui débite en continu une quantité constante, physiologiquement active pour un patient donné (la quantité de principes actifs délivrés étant fonction du poids du patient et de la dilution desdits principes actifs dans la solution injectée). Ce réservoir est relié à un cathéter de perfusion par un capillaire de très petit diamètre et de grande longueur, qui oppose une forte perte de charge au passage du fluide. Ledit capillaire est muni de moyens de contrôle permettant l'arrêt et la remise en route de l'écoulement, moyens comprenant une vanne télécommandée et des moyens de commande de ladite vanne. Des moyens de mesure du débit du liquide sont prévus à l'intérieur dudit capillaire.

L'invention sera mieux comprise à la lecture de la description qui va suivre, faite en référence aux dessins annexés, dans lesquels :
- la figure 1 est une représentation schématique de la chambre de transfert et des moyens de transfert du liquide vers le réservoir ;
- la figure 2 est une représentation schématique en section transversale d'un réservoir pressurisé selon un mode de réalisation particulier de l'invention ;
- la figure 3 est une représentation schématique d'un dispositif de perfusion implantable selon l'invention.

Comme illustré dans la figure 1, le dispositif 1 de l'invention comprend une chambre de transfert 2, implantée sous la peau du patient. L'orifice de remplissage de la chambre 2 est muni d'un septum 3, à travers lequel l'aiguille d'une seringue ou tout autre moyen approprié peut délivrer un médicament liquide au patient. Les moyens de transfert du liquide entre la chambre 2 et le réservoir qui permet de délivrer le médicament au patient comprennent le tube 4 et un clapet ou une valve anti-retour 5, installé sur le tube 4, de façon à permettre le passage du liquide uniquement vers ledit réservoir, dans le sens indiqué.

La figure 2 illustre un réservoir pressurisé permettant de délivrer un liquide au patient selon un mode de réalisation particulier de l'invention.

Le réservoir 6 comporte une enveloppe rigide 7 qui comprend une chambre à fluide à changement de phase 8, à l'intérieur de laquelle se trouve la chambre à médicament 9, délimitée par une membrane (poche) souple 10. Dans une variante de réalisation, le capillaire 11 servant à délivrer le médicament au patient est placé à l'intérieur de la chambre à médicament 9. Des moyens de remplissage 12 sont prévus pour permettre le remplissage en fluide à changement de phase avant la mise en service du réservoir 6.

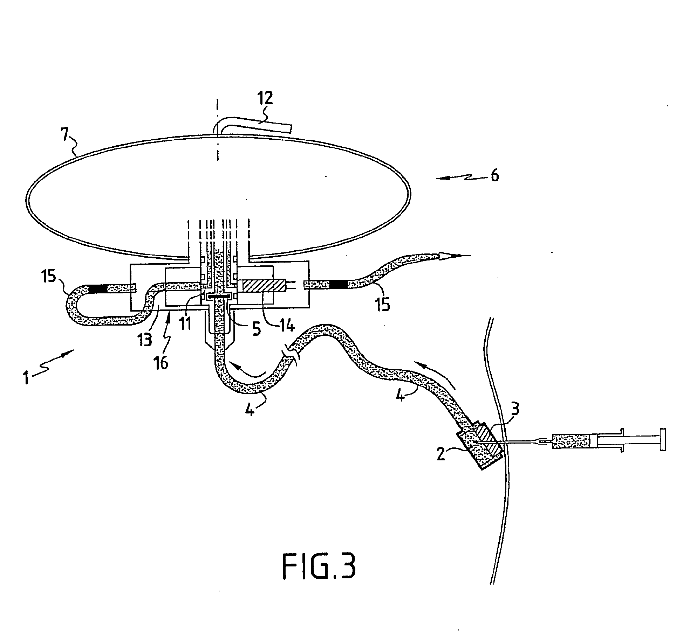

La figure 3 illustre les moyens de contrôle de l'écoulement du liquide dans le capillaire 11, comprenant notamment une vanne 13, ainsi que des moyens 14 permettant de mesurer le débit dans le capillaire 11.

L'alimentation en liquide se fait par le tube 4, à partir de la chambre 2. Le liquide à perfuser est injecté au patient à l'aide d'un cathéter 15, par son extrémité distale. Le cathéter 15 est relié au capillaire 11 au niveau de la sortie du réservoir 6.

Les différents éléments entrant dans la constitution du dispositif de l'invention seront décrits en détail ci-après.

### Chambre de transfert

La chambre de transfert 2 est implantée chirurgicalement dans les tissus cellulo-graisseux sous-cutanés du patient. Son boîtier étanche est muni, dans sa partie centrale, d'un septum 3, et latéralement, d'un tube de sortie. La chambre de transfert 2 est reliée au tube 4 réalisé en un matériau biocompatible (par exemple, en silicone ou en polyuréthane radio-opaque).

L'extrémité proximale du tube 4 est raccordée audit tube de sortie de la chambre 2. La connexion entre ledit tube de sortie et le tube 4 est assurée par une bague de sécurité (non représentée). L'extrémité distale dudit tube 4 vient se raccorder au réservoir à pression constante 6. Au niveau de la jonction entre le tube 4 et le réservoir 6 se trouve le clapet anti-retour 5.

Le volume interne de la chambre de transfert 2 est faible au regard de celui de la chambre à médicament 9 du réservoir 6 ; le rôle de la chambre de transfert 2 n'est pas de stocker le liquide à perfuser, mais de le transférer vers le réservoir 6 et de permettre ainsi le remplissage périodique de ladite chambre à médicament 9. Le médicament liquide à perfuser est injecté (après avoir été filtré sur des supports biocompatibles et dégazé), par exemple à l'aide d'une aiguille qui traverse le septum 3, débouche dans la chambre de transfert 2, d'où il passe dans le tube 4 et est ensuite stocké dans la chambre à médicament 9.

Afin d'éviter l'obstruction du tube 4, un filtre stérile est placé en sortie de la chambre de transfert 2, par exemple, au niveau dudit tube 4 de sortie de la chambre 2.

Le rinçage de la chambre de transfert 2 est possible, par exemple en utilisant deux aiguilles de huber en même temps. La première aiguille est connectée à une seringue qui injecte le liquide de nettoyage (par exemple, une solution diluée du liquide à administrer au patient). La deuxième aiguille reste libre et sert à évacuer le liquide de rinçage. La pression engendrée par l'aiguille qui injecte doit rester très inférieure à la pression du liquide contenu dans le réservoir 6.

### Réservoir permettant de délivrer le liquide au patient

Le réservoir 6 est implanté dans le corps du patient, à un endroit convenablement choisi pour son confort. Ledit réservoir peut être placé à une distance comprise entre 0,5 et 40 cm de la chambre de transfert 2.

Le réservoir 6 comporte une enveloppe rigide 7, réalisée dans un matériau biocompatible comme le titane, étanche au liquide à perfuser et aux fluides corporels.

Cette enveloppe correspond à une enceinte de 30 à 60 mm de diamètre, de 10 à 20 mm d'épaisseur et de masse totale (y compris la charge en liquide à perfuser et le capillaire) inférieure à 60 g.

Le réservoir 6 comprend une chambre 9 contenant le liquide à perfuser, connectée à un capillaire 11.

Le réservoir 6 peut délivrer ledit liquide au patient, tout en étant sous-pressurisé, auquel cas il est muni de moyens qui assurent un débit constant du liquide contenu dans la chambre 9 le long du capillaire 11. Dans un mode de réalisation particulier de l'invention, le réservoir 6 comprend des moyens de mise en pression constante du liquide contenu dans la chambre 9, selon les techniques connues par l'homme du métier : au moyen d'un ressort mécanique, par gaz comprimé, par évaporation d'un liquide à température constante.

La mise en pression du liquide permet à celui-ci de s'écouler dans le capillaire 11. En principe, il suffit d'une faible pression pour obtenir cet écoulement après que le capillaire 11 ait été complètement rempli de liquide. La pression au point d'injection peut varier en fonction de la position du patient et du possible développement d'un caillot autour du cathéter d'injection. Une pression comprise entre quelques bars à quelques dizaines de bars est suffisante pour compenser ces différences de pression.

Dans une variante préférée de réalisation, la pressurisation du réservoir 6 est réalisée en mettant en oeuvre un fluide à changement de phase. Dans ce cas, l'enveloppe rigide 7 est étanche audit fluide à changement de phase.

### Chambre à fluide à changement de phase

La chambre à fluide à changement de phase 8 contient un mélange liquide-vapeur d'un fluide à changement de phase, dont la pression ne dépend que de la température quasi constante du corps humain, environ 37±1°C. Cette pression est transmise au liquide à perfuser au travers d'une membrane souple et étanche 10 ; la pression du liquide à injecter devient ainsi elle-même constante et égale à celle du mélange liquide-vapeur. Comme propulseur peut être utilisé tout corps à changement de phase liquide-vapeur dont la tension de vapeur à 37°C est comprise entre quelques bar et quelques dizaines de bar. Dans un mode de réalisation, le gaz propulseur est l'isobutane, dont la tension de vapeur à 37°C est de 4,93 bar absolu. Dans une autre variante de réalisation, le gaz propulseur peut être le propane, dont la tension de vapeur est de 12,75 bar à 37°C. Quand la pressurisation du réservoir 6 se fait au moyen d'un fluide à changement de phase, l'injection du liquide dans la chambre de transfert 2 doit être réalisée suffisamment lentement pour que le changement de phase du fluide contenu dans le réservoir à pression constante 6 puisse se réaliser. Même dans ces conditions, le temps nécessaire pour remplir la chambre à médicament 9 via la chambre de transfert 2 reste inférieur à 10 min.

### Chambre à médicament

La chambre à médicament 9 a une contenance limitée, par exemple, à 10 ml, ce qui correspond à une autonomie d'utilisation de deux à quatre semaines, pour un débit fixe de 1 à 50 µl/h. D'une manière préférée, le volume mensuel à injecter est de 7 ml, ce qui correspond à un débit de 10 µl/h environ.

Dans une variante préférée de réalisation, la poche souple 9, contenant le médicament liquide à injecter et le capillaire en spire 11, est placée à l'intérieur de la chambre 8 contenant le fluide à changement de phase. Les avantages de cette solution sont nombreux :
- il n'y pas de contact entre le médicament liquide et l'enveloppe externe 7 de l'appareil, ce qui limite les risques de perçage de l'appareil ;
- il n'y a pas d'efforts localisés importants appliqués sur la membrane 10 ;
- la pression du médicament est effectivement égale à celle du fluide à changement de phase, puisque la poche 10 est très déformable ;
- le remplissage en fluide à changement de phase se fait après fermeture de l'enveloppe 7 ;
- le remplissage en médicament liquide se fait après fermeture de l'enveloppe 7;
- la membrane 10 empêche l'introduction de corps étrangers pouvant se former lors de la soudure ou de l'assemblage de l'enveloppe rigide 7 ;
- la surface de contact entre le fluide à changement de phase et les tissus qui entourent le réservoir 6 est à son maximum, optimisant ainsi le transfert d'énergie thermique entre ces tissus et le fluide à changement de phase, ce qui est important notamment lors du remplissage dudit réservoir avec un liquide dont la température, comprise entre 4 et 25°C environ, reste inférieure à celle de l'organisme. De ce fait, les variations de la pression du fluide à changement de phase lors du remplissage restent négligeables et n'induisent pas des variations importantes du débit du liquide à perfuser.

### Système de réglage de débit par mise en oeuvre d'une perte de charge contrôlée

Le contrôle du débit par tube capillaire s'effectue en mettant en oeuvre un réservoir 6 pressurisé contenant, dans la chambre 9, le liquide à injecter (comme décrit plus haut) et un tube capillaire en spire 11 parcouru par le liquide à injecter et fonctionnant en régime laminaire.

Les caractéristiques du capillaire 11, calculées à partir de plusieurs paramètres :
- l'ordre de grandeur du débit à injecter, par exemple voisin de 2,7 x 10⁻¹² m³/s pour de l'insuline à 100 unités (ce qui correspond à un débit mensuel d'environ 7 ml) ;
- la température de fonctionnement en intra-corporel, qui est proche de 37°C ;
- la viscosité dynamique du liquide à injecter ; elle correspond à 0,695 x 10⁻³ Pa.s, si on suppose que le liquide est assimilable à l'eau,
- la forme du capillaire (section circulaire, triangulaire ou rectangulaire), conduisent à des diamètres allant de 15 à 60 µm, pour des longueurs comprises entre 1 et 15 m et un pas de 100 à 150 µm entre deux éléments de spire successifs.

Dans une variante préférée de réalisation, le capillaire 11 présente une section triangulaire ou rectangulaire, l'espace capillaire étant créé par la mise en contact de deux plans (surfaces) dont l'un comporte une rainure. Cette solution apporte plus de sécurité au niveau du trajet et du débit du liquide à perfuser.

Dans ce cas, le capillaire en spire 11 peut présenter les caractéristiques suivantes : section carrée, environ 40 µm de côté, longueur comprise entre 12 à 15 m et un pas de spire de 120 µm.

La spire peut être réalisée en plusieurs matériaux, tels que les matériaux plastiques biocompatibles ou le silicium.

Avant mise en service, la spire 11 doit être étalonnée, cette opération pouvant être effectuée avec un liquide (par exemple, de l'eau ou le médicament à perfuser) ou au moyen d'un gaz comprimé.

La constance du débit est assurée par la température quasi constante du corps du patient ; les variations de températures entraînent des variations de débit inférieures à 2% par degré Celsius, et sont compatibles avec les variations des besoins de l'utilisateur.

Le dispositif 1 de l'invention est muni de moyens de contrôle 16 permettant d'arrêter l'écoulement du liquide dans le capillaire 11 et de le redémarrer ultérieurement. Lesdits moyens de contrôle comprennent :
- une vanne 13 télécommandée,
- des moyens de commande de la vanne (non représentés), qui peuvent être actionnés soit en réponse aux données recueillies par un capteur (par exemple, un capteur de glycémie chez les patients diabétiques, ledit capteur étant interne ou externe), soit suite à une intervention manuelle commandée de l'extérieur. Pour arrêter ou redémarrer le système à partir d'ordres externes, peut être utilisée une commande avec transmission sans fil, par exemple par radio.

Cette manoeuvre d'arrêt et de mise en service intervient en cas de problèmes (hypoglycémie momentanée) ou d'opérations spécifiques.

La consommation électrique de la vanne 13 et de son système de commande peut être assurée par une pile électrique convenablement dimensionnée ou par un accumulateur rechargeable de l'extérieur au moyen d'ondes radioélectriques.

Une vanne électrique miniaturisée, pouvant correspondre aux besoins du dispositif de l'invention, est représentée, par exemple, par l'électrovanne EPSV commercialisée par la société américaine Lee Company, à laquelle est ajouté un aimant, de façon à la faire fonctionner à partir d'impulsions électriques de courte durée, la vanne ne consommant pas de courant en position ouverte ou fermée.

Le capillaire 11 comprend également des moyens de mesure du débit 14, consistant en un capteur de pression pré-étalonné à point central et en son conditionneur. Ledit capteur mesure la pression du liquide en un point situé entre l'entrée et la sortie du capillaire 11, préférablement en son milieu. En fonctionnement normal, la pression est égale à une valeur comprise entre la pression du liquide dans le réservoir et la pression au point d'injection, proche de la pression atmosphérique. En l'absence du débit, la pression mesurée s'établit à l'une ou l'autre desdites valeurs, selon la partie du conduit qui est obstruée. Le capteur de pression permet donc d'indiquer la présence d'un débit et d'un mesurer précisément la valeur en temps réel.

Cette mesure est faite à partir de la viscosité du liquide à injecter, de certaines caractéristiques du capillaire (pressions amont et aval du capillaire) et de la température du corps humain.

Le capteur de pression est un appareil formé d'un corps et d'une membrane dont on mesure la déformation au moyen de jauges de contrainte. De tels capteurs, adaptés à une utilisation dans le cadre du dispositif de l'invention, sont, par exemple, les capteurs miniatures commercialisés par la compagnie américaine ENTRAN.

Le système de contrôle-commande du dispositif de l'invention comprend donc deux fonctions :
- la commande de la vanne 13 à partir d'ordres internes ou externes (ouverte ou fermé),
- l'émission d'un signal d'alerte en cas de dysfonctionnement détecté par le capteur de pression,
la transmission dans les deux cas pouvant se faire par radio.

### Cathéter

Le cathéter 15 est implanté dans une voie sanguine, en particulier une veineuse centrale, de préférence dans une zone à forte turbulence, de façon à éviter la formation d'amas de fibrine à son extrémité. Ce cathéter permet la liaison entre le réservoir 6 et le point d'injection.

Un colmatage du cathéter peut se produire si la période d'arrêt du débit par ledit cathéter dépasse une durée de temps d'environ 30 min. Afin d'éviter toute obstruction du cathéter 15, des moyens de surveillance du débit dans le capillaire 11 sont intégrés au niveau des moyens de commande de la vanne 13, permettant de redémarrer ladite vanne à un intervalle régulier prédéfini pendant une durée prédéfinie; concrètement, elle consiste à installer, au niveau des moyens de commande de la vanne 13, un programme (des instructions implémentées dans un circuit intégré) qui prévoit, lorsque la vanne 13 est en position d'arrêt, un débit de courte durée (30 s à 1 min) qui redémarre toutes les 10 min environ.

Le cathéter 15 doit fonctionner pendant de longues périodes, allant de 5 à 7 ans ; il est donc impératif qu'il soit correctement fixé au niveau de la voie veineuse centrale choisie, qu'il ne puisse se détacher et qu'il ne forme pas de caillot dont la perte de charge propre pourrait être élevée et conduire à une diminution de la différence de pression aux extrémités du circuit.

La dose de médicament à injecter peut être variée en fonction du poids du patient, par exemple en utilisant les capillaires ayant les coefficients de débit les plus élevés pour les patients les plus corpulents et en diluant le médicament pour obtenir des doses adaptées.

Dans un mode de réalisation, la quantité de liquide à perfuser pourra être globalement modifiée par la variation du temps d'ouverture de la vanne 13, selon des programmes prédéfinis, en fonction des besoins spécifiques du patient.

Le dispositif de l'invention peut être complémentaire à d'autres moyens existants utilisés pour baisser la glycémie : injections, inhalations, etc d'insuline.

## Revendications (Revendications pour l'(les) Etat(s) contractant(s) suivant(s): DE, FR, GB, IT)

1. Dispositif implantable pour administration d'un liquide, notamment un médicament, chez l'homme ou l'animal, comprenant un réservoir (6) permettant de délivrer ledit liquide et destiné à être placé dans une cavité du corps du patient, ledit réservoir comprend une entrée prévue pour ledit liquide et au moins une sortie pour délivrer ledit liquide au patient, **caractérisé en ce qu'**il comprend:
- une chambre de transfert (2) permettant l'alimentation dudit réservoir avec ledit liquide, apte à être implantée sous la peau, ladite chambre de transfert (2) comprend un orifice de remplissage muni d'un septum (3), à travers lequel tout moyen approprié peut délivrer ledit liquide,
- des moyens de transfert dudit liquide entre ladite chambre de transfert (2) et ledit réservoir (6), et
- un filtre stérile placé en sortie de la chambre de transfert (2).

2. Dispositif selon la revendication **1,** dans lequel ladite sortie du réservoir (6), prévue pour délivrer un liquide au patient, est reliée à un capillaire (11).

3. Dispositif selon la revendication **2,** dans lequel le capillaire (11) est muni de moyens de contrôle (16) permettant de régler le débit du liquide dans ledit capillaire (11).

4. Dispositif selon la revendication **3,** dans lequel lesdits moyens de contrôle (16) comprennent une vanne (13) télécommandée et des moyens de commande de ladite vanne.

5. Dispositif selon la revendication **4,** dans lequel lesdits moyens de commande de la vanne (13) comprennent un capteur interne ou externe.

6. Dispositif selon l'une quelconque des revendications **2 à 5,** dans lequel le capillaire (11) est muni d'un cathéter (15) à son extrémité distale.

7. Dispositif selon l'une des revendications **2 à 6,** dans lequel le capillaire (11) est muni de moyens de mesure du débit (14) du liquide dans ledit capillaire.

8. Dispositif selon la revendication **7,** dans lequel lesdits moyens de mesure du débit (14) comprennent un capteur de pression à point central.

9. Dispositif selon l'une quelconque des revendications **1 à 7,** dans lequel ledit réservoir (6) comprend des moyens de mise en pression constante du liquide contenu dans la chambre (9).

10. Dispositif selon la revendication **9,** dans lequel lesdits moyens de mise en pression constante du liquide contenu dans la chambre (9) comprennent l'utilisation d'un fluide à changement de phase.

11. Dispositif selon la revendication **10,** dans lequel le fluide à changement de phase est l'isobutane ou le propane.

12. Dispositif selon l'une quelconque des revendications **1 à 11,** dans lequel lesdits moyens de transfert du liquide entre la chambre de transfert (2) et le réservoir (6) comprennent un tube (4) et un clapet (5).

13. Dispositif selon une quelconque des revendications **1 à 12,** dans lequel ledit tube (4) présente une longueur qui permet d'implanter la chambre de transfert (2) sous la peau et le réservoir (6) à distance, à un endroit choisi du corps du patient.

14. Dispositif selon la revendication **4,** dans lequel on prévoit des moyens de surveillance du débit dans le capillaire (11), intégrés au niveau des moyens de commande de la vanne (13), permettant de redémarrer la vanne à un intervalle régulier prédéfini pendant une durée prédéfinie.

## Revendications (Revendications pour l'(les) Etat(s) contractant(s) suivant(s): AT, BE, CH, DK, ES, BR, LI, LU, MC, NL, SE)

1. Dispositif implantable pour administration d'un liquide, notamment un médicament, chez l'homme ou l'animal, comprenant un réservoir (6) permettant de délivrer ledit liquide et destiné à être placé dans une cavité du corps du patient, ledit réservoir comprend une entrée prévue pour ledit liquide et au moins une sortie pour délivrer ledit liquide au patient, **caractérisé en ce qu'**il comprend:
- une chambre de transfert (2) permettant l'alimentation dudit réservoir avec ledit liquide, apte à être implantée sous la peau, ladite chambre de transfert (2) comprend un orifice de remplissage muni d'un septum (3), à travers lequel tout moyen approprié peut délivrer ledit liquide, et
- des moyens de transfert dudit liquide entre ladite chambre de transfert (2) et ledit réservoir (6).

2. Dispositif selon la revendication **1,** dans lequel ladite sortie du réservoir (6), prévue pour délivrer un liquide au patient, est reliée à un capillaire (11).

3. Dispositif selon la revendication **2,** dans lequel le capillaire (11) est muni de moyens de contrôle (16) permettant de régler le débit du liquide dans ledit capillaire (11).

4. Dispositif selon la revendication **3,** dans lequel lesdits moyens de contrôle (16) comprennent une vanne (13) télécommandée et des moyens de commande de ladite vanne.

5. Dispositif selon la revendication **4,** dans lequel lesdits moyens de commande de la vanne (13) comprennent un capteur interne ou externe.

6. Dispositif selon l'une quelconque des revendications **2 à 5,** dans lequel le capillaire (11) est muni d'un cathéter (15) à son extrémité distale.

7. Dispositif selon l'une des revendications **2 à 6,** dans lequel le capillaire (11) est muni de moyens de mesure du débit (14) du liquide dans ledit

8. Dispositif selon la revendication **7,** dans lequel lesdits moyens de mesure du débit (14) comprennent un capteur de pression à point central.

9. Dispositif selon l'une quelconque des revendications **1 à 7,** dans lequel ledit réservoir (6) comprend des moyens de mise en pression constante du liquide contenu dans la chambre (9).

10. Dispositif selon la revendication **9,** dans lequel lesdits moyens de mise en pression constante du liquide contenu dans la chambre (9) comprennent l'utilisation d'un fluide à changement de phase.

11. Dispositif selon la revendication **10,** dans lequel le fluide à changement de phase est l'isobutane ou le propane.

12. Dispositif selon l'une quelconque des revendications **1 à 11,** dans lequel lesdits moyens de transfert du liquide entre la chambre de transfert (2) et le réservoir (6) comprennent un tube (4) et un clapet (5).

13. Dispositif selon une quelconque des revendications **1 à 12,** dans lequel ledit tube (4) présente une longueur qui permet d'implanter la chambre de transfert (2) sous la peau et le réservoir (6) à distance, à un endroit choisi du corps du patient.

14. Dispositif selon la revendication **4,** dans lequel on prévoit des moyens de surveillance du débit dans le capillaire (11), intégrés au niveau des moyens de commande de la vanne (13), permettant de redémarrer la vanne à un intervalle régulier prédéfini pendant une durée prédéfinie.

## Patentansprüche (Patentansprüche für folgende(n) Vertragsstaat(en): DE, FR, GB, IT)

1. Implantierbare Vorrichtung zur Verabreichung einer Flüssigkeit, insbesondere eines Medikaments, an einen Menschen oder ein Tier, umfassend einen Behälter (6), der es ermöglicht, die Flüssigkeit abzugeben und der dazu bestimmt ist, in einem Hohlraum des Körpers eines Patienten angeordnet zu werden, wobei der Behälter einen Einlass, der für die Flüssigkeit vorgesehen ist, und mindestens einen Auslass, um die Flüssigkeit an den Patienten abzugeben, aufweist, **dadurch gekennzeichnet, dass** sie umfasst:
- eine Überführungskammer (2), die das Versorgen des Behälters mit der Flüssigkeit ermöglicht und geeignet ist, unter die Haut implantiert zu werden, wobei die Überführungskammer (2) eine Einfüllöffnung aufweist, die mit einem Septum (3) versehen ist, durch die jedes geeignete Mittel die Flüssigkeit abgeben kann,
- Mittel zum Überführen der Flüssigkeit zwischen der Überführungskammer (2) und dem Behälter (6) und
- einen sterilen Filter, der an dem Auslass der Überführungskammer (2) angeordnet ist.

2. Vorrichtung nach Anspruch 1, wobei der Auslass des Behälters (6), der dazuvorgesehen ist, eine Flüssigkeit an den Patienten abzugeben, mit einer Kapillare (11) verbunden ist.

3. Vorrichtung nach Anspruch 2, wobei die Kapillare (11) mit Kontrollmitteln (16) versehen ist, die es ermöglichen, den Durchsatz der Flüssigkeit in der Kapillare (11) zu regeln.

4. Vorrichtung nach Anspruch 3, wobei die Kontrollmittel (16) ein ferngesteuertes Ventil (13) und Mittel zum Steuern des Ventils aufweisen.

5. Vorrichtung nach Anspruch 4, wobei die Mittel zum Steuern des Ventils (13) einen internen oder externen Sensor aufweisen.

6. Vorrichtung nach einem der Ansprüche 2 bis 5, wobei die Kapillare (11) an ihrem distalen Ende mit einem Katheter (15) versehen ist.

7. Vorrichtung nach einem der Ansprüche 2 bis 6, wobei die Kapillare (11) mit Mitteln zum Messen des Durchsatzes (14) der Flüssigkeit in der Kapillare versehen ist.

8. Vorrichtung nach Anspruch 7, wobei die Mittel zum Messen des Durchsatzes (14) einen Drucksensor im Mittelpunkt aufweisen.

9. Vorrichtung nach einem der Ansprüche 1 bis 7, wobei der Behälter (6) Mittel zum konstanten Druckaufbauen der Flüssigkeit, die in der Kammer (9) enthalten ist, aufweist.

10. Vorrichtung nach Anspruch 9, wobei die Mittel zum konstanten Druckaufbauen der Flüssigkeit, die in der Kammer (9) enthalten ist, das Verwenden einer Phasenwechselflüssigkeit aufweisen.

11. Vorrichtung nach Anspruch 10, wobei die Phasenwechselflüssigkeit Isobutan oder Propan ist.

12. Vorrichtung nach einem der Ansprüche 1 bis 11, wobei die Mittel zum Überführen der Flüssigkeit zwischen der Überführungskammer (2) und dem Behälter (6) einen Schlauch (4) und ein Ventil (5) aufweisen.

13. Vorrichtung nach einem der Ansprüche 1 bis 12, wobei der Schlauch (4) eine Länge aufweist, die es ermöglicht, die Überführungskammer (2) unter die Haut und den Behälter (6) in einem Abstand an einer ausgewählten Stelle des Körper des Patienten zu implantieren.

14. Vorrichtung nach Anspruch 4, wobei Mittel zum Überwachen des Durchsatzes in der Kapillare (11) vorgesehen und auf der Ebene der Mittel zum Steuern des Ventils (13) integriert sind und es ermöglichen, das Ventil in einem regelmäßigen Abstand während einer vordefinierten Dauer neu zu starten.

## Patentansprüche (Patentansprüche für folgende(n) Vertragsstaat(en): AT, BE, CH, DK, ES, BR, LI, LU, MC, NL, SE)

1. Implantierbare Vorrichtung zur Verabreichung einer Flüssigkeit, insbesondere eines Medikaments, an einen Menschen oder ein Tier, umfassend einen Behälter (6), der es ermöglicht, die Flüssigkeit abzugeben und der dazu bestimmt ist, in einem Hohlraum des Körpers eines Patienten angeordnet zu werden, wobei der Behälter einen Einlass, der für die Flüssigkeit vorgesehen ist, und mindestens einen Auslass, um die Flüssigkeit an den Patienten abzugeben, aufweist, **dadurch gekennzeichnet, dass** sie umfasst:
- eine Überführungskammer (2), die das Versorgen des Behälters mit der Flüssigkeit ermöglicht und geeignet ist, unter die Haut implantiert zu werden, wobei die Überführungskammer (2) eine Einfüllöffnung aufweist, die mit einem Septum (3) versehen ist, durch die jedes geeignete Mittel die Flüssigkeit abgeben kann, und
- Mittel zum Überführen der Flüssigkeit zwischen der Überführungskammer (2) und dem Behälter (6).

2. Vorrichtung nach Anspruch 1, wobei der Auslass des Behälters (6), der dazuvorgesehen ist, eine Flüssigkeit an den Patienten abzugeben, mit einer Kapillare (11) verbunden ist.

3. Vorrichtung nach Anspruch 2, wobei die Kapillare (11) mit Kontrollmitteln (16) versehen ist, die es ermöglichen, den Durchsatz der Flüssigkeit in der Kapillare (11) zu regeln.

4. Vorrichtung nach Anspruch 3, wobei die Kontrollmittel (16) ein ferngesteuertes Ventil (13) und Mittel zum Steuern des Ventils aufweisen.

5. Vorrichtung nach Anspruch 4, wobei die Mittel zum Steuern des Ventils (13) einen internen oder externen Sensor aufweisen.

6. Vorrichtung nach einem der Ansprüche 2 bis 5, wobei die Kapillare (11) an ihrem distalen Ende mit einem Katheter (15) versehen ist.

7. Vorrichtung nach einem der Ansprüche 2 bis 6, wobei die Kapillare (11) mit Mitteln zum Messen des Durchsatzes (14) der Flüssigkeit in der Kapillare versehen ist.

8. Vorrichtung nach Anspruch 7, wobei die Mittel zum Messen des Durchsatzes (14) einen Drucksensor im Mittelpunkt aufweisen.

9. Vorrichtung nach einem der Ansprüche 1 bis 7, wobei der Behälter (6) Mittel zum konstanten Druckaufbauen der Flüssigkeit, die in der Kammer (9) enthalten ist, aufweist.

10. Vorrichtung nach Anspruch 9, wobei die Mittel zum konstanten Druckaufbauen der Flüssigkeit, die in der Kammer (9) enthalten ist, das Verwenden einer Phasenwechselflüssigkeit aufweisen.

11. Vorrichtung nach Anspruch 10, wobei die Phasenwechselflüssigkeit Isobutan oder Propan ist.

12. Vorrichtung nach einem der Ansprüche 1 bis 11, wobei die Mittel zum Überführen der Flüssigkeit zwischen der Überführungskammer (2) und dem Behälter (6) einen Schlauch (4) und ein Ventil (5) aufweisen.

13. Vorrichtung nach einem der Ansprüche 1 bis 12, wobei der Schlauch (4) eine Länge aufweist, die es ermöglicht, die Überführungskammer (2) unter die Haut und den Behälter (6) in einem Abstand an einer ausgewählten Stelle des Körper des Patienten zu implantieren.

14. Vorrichtung nach Anspruch 4, wobei Mittel zum Überwachen des Durchsatzes in der Kapillare (11) vorgesehen und auf der Ebene der Mittel zum Steuern des Ventils (13) integriert sind und es ermöglichen, das Ventil in einem regelmäßigen Abstand während einer vordefinierten Dauer neu zu starten.

## Claims (Claims for the following Contracting State(s): DE, FR, GB, IT)

1. An implantable device for administering a liquid, in particular a medication, in man or animal, comprising a supply (6) enabling said liquid to be delivered and suitable for being placed in a cavity of the body of the patient, said supply comprises an inlet for receiving said liquid and at least one outlet for delivering said liquid to the patient,
**characterized in that** the device comprises:
• a transfer chamber (2) enabling the supply to be fed with said liquid, configured to be implanted under the skin, said transfer chamber (2) comprises a filler orifice provided with a septum (3) through which any suitable means can deliver said liquid,
• transfer means for transferring said liquid between the transfer chamber (2) and said supply (6); and
• a sterile filter placed at the outlet from the transfer chamber (2).

2. A device according to claim 1, in which said outlet of the supply (6) for delivering a liquid to the patient is connected to a capillary (11).

3. A device according to claim 2, in which the capillary (11) is provided with control means (16) enabling the flow of liquid in said capillary (11) to be adjusted.

4. A device according to claim 3, in which said control means (16) comprise a remotely-controlled valve (13) and means for controlling said valve.

5. A device according to claim 4, in which said means for controlling the valve (13) comprise an internal or external sensor.

6. A device according to any one of claims 2 to 5, in which the capillary (11) is provided with a catheter (15) at its distal end.

7. A device according to any one of claims 2 to 6, in which the capillary (11) is provided with means (14) for measuring the flow of the liquid in said capillary.

8. A device according to claim 7, in which said flow measurement means (14) comprise a central point pressure sensor.

9. A device according to any one of claims 1 to 7, in which said supply (6) includes means for putting the liquid contained in the chamber (9) to a constant pressure.

10. A device according to claim 9, in which said means for putting the liquid contained in the chamber (9) to a constant pressure comprise using a phase-change fluid.

11. A device according to claim 10, in which the phase-change fluid is isobutane or propane.

12. A device according to any one of claims 1 to 11, in which said means for transferring liquid between the transfer chamber (2) and the supply (6) comprise a tube (4) and a check valve (5).

13. A device according to any one of claims 1 to 12, in which said tube (4) presents a length that enables the transfer chamber (2) to be implanted under the skin and the supply (6) at a distance therefrom, at a selected location in the body of the patient.

14. A device according to claim 4, in which means are provided for monitoring the flow in the capillary (11), integrated with the means for controlling the valve (13), enabling the valve to be restarted at predefined regular intervals for predefined durations.

## Claims (Claims for the following Contracting State(s): AT, BE, CH, DK, ES, BR, LI, LU, MC, NL, SE)

1. An implantable device for administering a liquid, in particular a medication, in man or animal, comprising a supply (6) enabling said liquid to be delivered and suitable for being placed in a cavity of the body of the patient, said supply comprises an inlet for receiving said liquid and at least one outlet for delivering said liquid to the patient,
**characterized in that** the device comprises:
• a transfer chamber (2) enabling the supply to be fed with said liquid, configured to be implanted under the skin, said transfer chamber (2) comprises a filler orifice provided with a septum (3) through which any suitable means can deliver said liquid, and
• transfer means for transferring said liquid between the transfer chamber (2) and said supply (6).

2. A device according to claim 1, in which said outlet of the supply (6) for delivering a liquid to the patient is connected to a capillary (11).

3. A device according to claim 2, in which the capillary (11) is provided with control means (16) enabling the flow of liquid in said capillary (11) to be adjusted.

4. A device according to claim 3, in which said control means (16) comprise a remotely-controlled valve (13) and means for controlling said valve.

5. A device according to claim 4, in which said means for controlling the valve (13) comprise an internal or external sensor.

6. A device according to any one of claims 2 to 5, in which the capillary (11) is provided with a catheter (15) at its distal end.

7. A device according to any one of claims 2 to 6, in which the capillary (11) is provided with means (14) for measuring the flow of the liquid in said capillary.

8. A device according to claim 7, in which said flow measurement means (14) comprise a central point pressure sensor.

9. A device according to any one of claims 1 to 7, in which said supply (6) includes means for putting the liquid contained in the chamber (9) to a constant pressure.

10. A device according to claim 9, in which said means for putting the liquid contained in the chamber (9) to a constant pressure comprise using a phase-change fluid.

11. A device according to claim 10, in which the phase-change fluid is isobutane or propane.

12. A device according to any one of claims 1 to 11, in which said means for transferring liquid between the transfer chamber (2) and the supply (6) comprise a tube (4) and a check valve (5).

13. A device according to any one of claims 1 to 12, in which said tube (4) presents a length that enables the transfer chamber (2) to be implanted under the skin and the supply (6) at a distance therefrom, at a selected location in the body of the patient.

14. A device according to claim 4, in which means are provided for monitoring the flow in the capillary (11), integrated with the means for controlling the valve (13), enabling the valve to be restarted at predefined regular intervals for predefined durations.
